# EUROPEAN PATENT SPECIFICATION

(11) **EP 4 056 543 B1**
(45) Date of publication and mention of the grant of the patent: **26.11.2025**
(21) Application number: 21214438.0
(22) Date of filing: 14.12.2021
(51) Int. Cl.: C05F 17/70, C05F 17/929, C05F 17/943

(54) **COMPOSTING APPARATUS AND METHOD OF PROCESSING ORGANIC MATTER**
KOMPOSTIERVORRICHTUNG UND VERFAHREN ZUR VERARBEITUNG VON ORGANISCHEM MATERIAL
APPAREIL DE COMPOSTAGE ET PROCÉDÉ DE TRAITEMENT DE MATIÈRE ORGANIQUE

(30) Priority: 10.03.2021 FI 20215254
(43) Date of publication of application: 14.09.2022
(73) Proprietor: Sineco Oy, 02150 Espoo (FI)
(72) Inventor: VARALTA, Federico, 02320 ESPOO (FI)
(74) Representative: Berggren Oy

(56) References cited:
- WO-A1-2014/167098
- WO-A1-2017/048124
- US-A- 5 451 523

## Description

### Technical field

The invention relates to a composting apparatus for processing organic matter, as defined in claim 1. The invention also concerns a method of processing organic matter in accordance with claim 12.

### Background

Organic waste, such as waste originating from food processing industry, animal manure and bedding, other organic matter originating from industry or agriculture, or leftover food collected from households and restaurants, can be composted in order to degrade the organic matter. Composting is an aerobic process, where organic material is decomposed into simpler organic and inorganic compounds by microorganisms. Composting takes place naturally even in uncovered stacks of organic material, but the process can be significantly faster in a composting apparatus that is provided with a closed vessel, where the parameters of the composting process can be monitored.

The rate of the composting process significantly depends on the conditions in the vessel of the composting apparatus. The main factors affecting the rate of the process are the moisture, particle size, oxygen concentration and temperature. If the parameters are not properly balanced, the metabolic rate of the microorganisms is slower and it affects the process efficiency. The process is exothermic as it generates heat together with carbon dioxide as a result of the reactions.

Composting apparatuses that are cylindrical and rotatable and comprise means for aeration are already known from WO2017/048124 and WO2014/167098.

### Summary

An object of the present invention is to provide an improved composting apparatus for processing organic matter. The characterizing features of the composting apparatus according to the invention are given in claim 1. Another object of the invention is to provide an improved method of processing organic matter. The characterizing features of the method according to the invention are given in claim 12.

The composting apparatus according to the invention comprises
- a cylindrical vessel for receiving and processing the organic matter, the cylindrical vessel having a first end and a second end and being rotatable about its longitudinal axis,
- a feeding opening arranged at the first end of the vessel for feeding the organic matter to be processed into the vessel,
- a discharge opening arranged at the second end of the vessel for discharging the processed organic matter from the vessel,
- transfer and mixing elements arranged inside the vessel and being configured to move the organic matter inside the vessel towards the second end of the vessel and to mix the organic matter when the vessel is rotated, and
- air supply means for supplying air into the organic matter to be processed at an aeration zone of the vessel,
characterised in that the aeration zone extends in the circumferential direction of the vessel over an angle of at least 120 degrees and in the longitudinal direction of the vessel from the first end of the vessel to a distance corresponding to 35-65 percent of the total length of the vessel.

The method according to the invention for processing organic matter using a composting apparatus defined above comprises
- a feeding step, in which organic matter is fed into the vessel,
- a composting step, in which the vessel is kept stationary for allowing composting of the organic matter,
- a moving step, in which the vessel is rotated for moving the organic matter towards the second end of the vessel, and
- a discharging step, in which the organic matter is discharged from the vessel.

With the composting apparatus and the method according to the invention, effective and uniform composting process can be achieved.

According to the invention, the aeration zone extends in the circumferential direction of the vessel over an angle of at least 120 degrees. Preferably, the aeration zone extends over at least 180 degrees. This allows uniform air supply into the organic matter.

According to the invention, the aeration zone extends in the longitudinal direction of the vessel from the first end of the vessel to a distance corresponding to 35-65 percent of the total length of the vessel. Approximately one to two thirds of the vessel is thus reserved for a primary composting phase, in which the organic matter is effectively aerated. However, the composting process can still continue in the remaining part of the vessel before the processed organic matter is discharged from the vessel.

According to an embodiment of the invention, the air supply means comprise a plurality of air pipes arranged within the vessel, each air pipe comprising a plurality of air outlets. By providing the vessel with several air pipes, the air is evenly distributed into the organic matter.

According to an embodiment of the invention, the air pipes extend in the longitudinal and/or circumferential direction of the vessel. This allows the air pipes to be easily connected to a common air distribution element, through which air can be supplied into the air pipes.

According to an embodiment of the invention, the air pipes are elevated 10-100 mm from the inner surface of the vessel. This allows the air to effectively penetrate into the organic matter.

According to an embodiment of the invention, the air supply means comprise an air blower. An air blower ensures effective and controllable air supply.

According to an embodiment of the invention, the apparatus comprises means for controlling discharging of air from the vessel. With the means for controlling the discharging of air, the humidity and/or the temperature within the vessel can be regulated.

According to an embodiment of the invention, the means for controlling the discharging of air comprise an air fan. The air fan ensures effective discharging of air.

According to an embodiment of the invention, the apparatus comprises feeding means for feeding the organic matter to be processed into the vessel. According to an embodiment of the invention, the apparatus comprises discharge means for discharging the processed organic matter from the vessel. The feeding and discharge means allow automated operation of the apparatus. The means can be conveyors, such as screw conveyors.

According to an embodiment of the invention, the apparatus comprises temperature measuring means for measuring temperature within the vessel and humidity measuring means for measuring air humidity within the vessel, and the apparatus is configured to control air supply via the air supply means and/or discharging of air from the vessel on the basis of at least one the temperature value and at least one humidity value measured within the vessel. This allows a target temperature to be reached rapidly and helps keeping the temperature and humidity at a desired level.

According to an embodiment of the invention, the composting step comprises at least one sub-step, during which temperature and humidity in the vessel is monitored, and air supply into the vessel via the air supply means and/or discharging of air from the vessel is controlled based on the monitored temperature and the monitored humidity. By controlling the air supply and the air discharge on the basis of the monitored temperature and humidity, a target temperature can be reached quickly and the temperature and humidity can be effectively maintained at a target level.

### Brief description of the drawings

Embodiments of the invention are described below in more detail with reference to the accompanying drawings, in which
Fig. 1 shows a perspective view of a composting apparatus according to an embodiment of the invention,
Fig. 2 shows a schematic view of a composting apparatus according to an embodiment of the invention,
Fig. 3 shows a side view of the composting apparatus of figure 1,
Fig. 4 shows a cross-sectional view of the composting apparatus of figure 3 taken along line C-C,
Fig. 5 shows an end view of the composting apparatus of figure 1,
Fig. 6 shows a cross-sectional view of the composting apparatus of figure 5 taken along line A-A,
Fig. 7 shows as a flowchart the method according to the invention,
Fig. 8 shows a view of an air pipe of the composting apparatus of figure 1,
Fig. 9 shows an enlarged view of part of the air pipe of figure 8,
Fig. 10 shows a composting apparatus according to another embodiment of the invention, and
Fig. 11 shows a partly cut view of the composting apparatus of figure 10.

### Detailed description

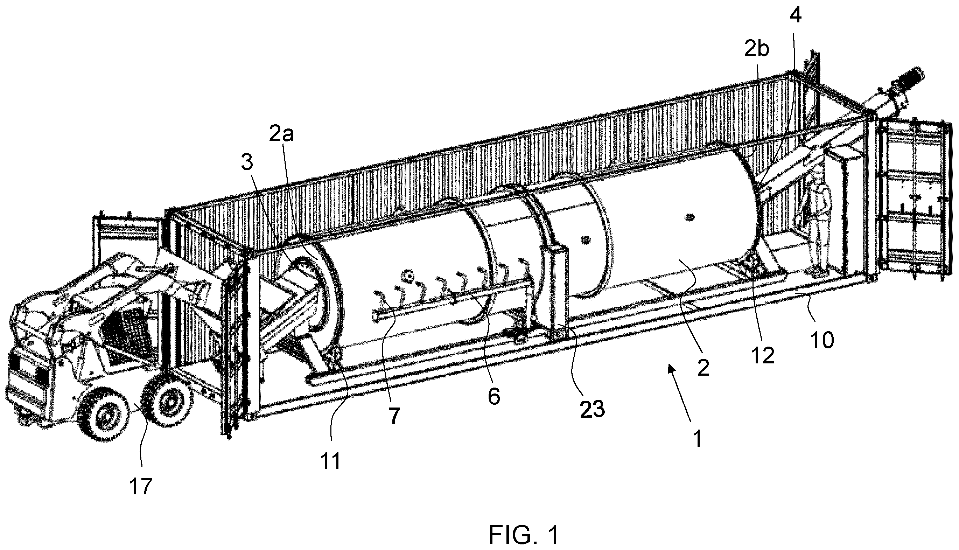

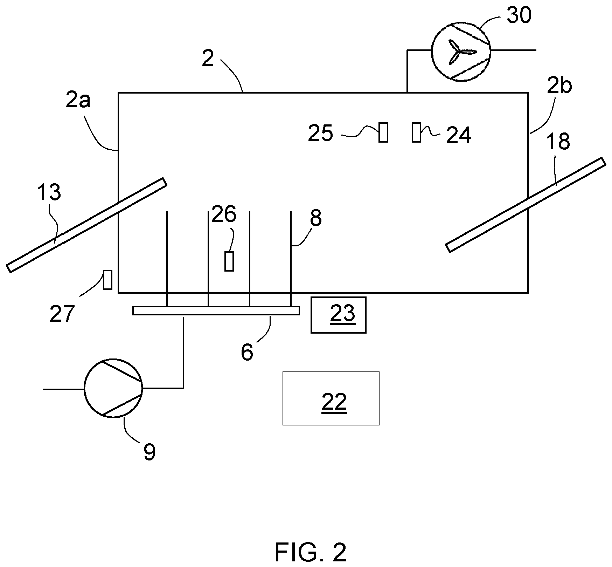

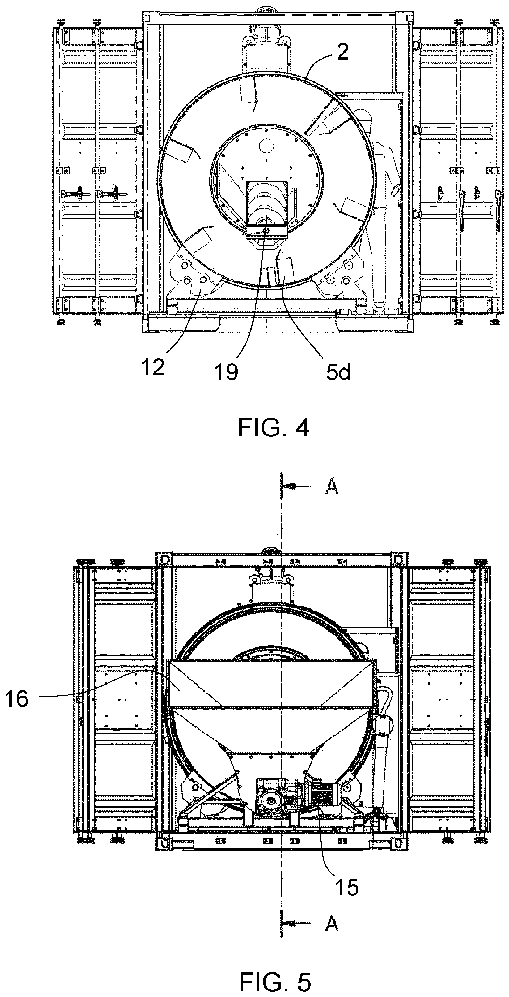

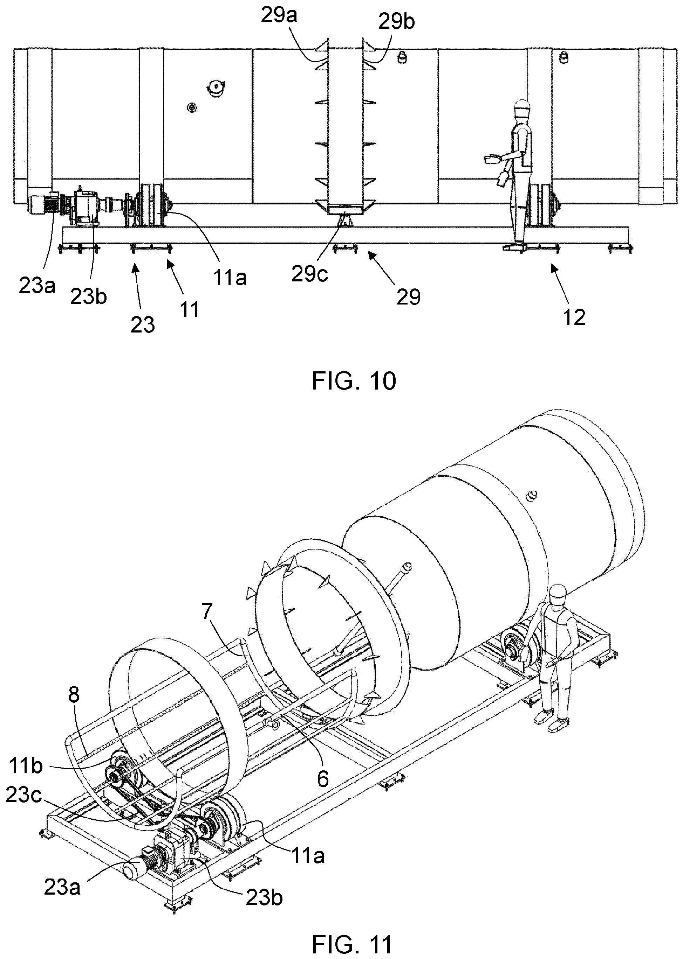

Figure 1 shows a composting apparatus 1 according to an embodiment of the invention. Figure 2 shows a schematic view of the composting apparatus of figure 1. Some details of the composting apparatus of figure 1 can be seen only in figures 2-6, 8 and 9. The apparatus shown in figures 1-6, 8 and 9 is hereinafter also referred to as a first embodiment. Figures 10 and 11 show a composting apparatus according to another embodiment of the invention. The apparatus of figures 10 and 11 is hereinafter also referred to as a second embodiment. The embodiment of figures 10 and 11 is similar to the embodiment of the other figures, and the description below is therefore applicable to both embodiments, unless otherwise stated. The features of the two embodiments could also be combined.

In the embodiment of figure 1, the composting apparatus 1 is arranged in a container 10. In figure 1, the roof and one of the side walls of the container 10 have been removed to show the features of the composting apparatus 1 itself. The container 10 is a standardized 40-foot intermodal container. In the embodiment of the figures, the composting apparatus 1 is configured to be arrangeable in a 40-foot container. However, it is not necessary to arrange the composting apparatus 1 in a container 10 and the composting apparatus 1 could also be dimensioned differently according to the needed capacity.

The composting apparatus 1 is configured to process organic matter under aerobic conditions, i.e. to compost the organic matter. In the composting process, organic material is decomposed into simpler organic and inorganic compounds by microorganisms. The organic matter to be processed can be any material suitable for composting. The organic matter can be, for instance, waste originating from food processing industry, animal manure and bedding, other organic matter originating from industry or agriculture, or leftover food collected from households and restaurants.

The composting apparatus comprises a vessel 2. The vessel 2 is a cylindrical tank. The vessel 2 could also be referred to as a drum. The vessel 2 can be made of steel. The vessel 2 can be a welded structure. The vessel 2 has a first end 2a and a second end 2b. The organic matter to be processed is fed into the vessel 2 at the first end of the vessel 2 and the processed matter is removed from the vessel 2 at the second end 2b of the vessel 2. The first end 2a could thus be referred to as a front end and the second end 2b could be referred to as a rear end. The vessel 2 is configured to be rotatable about its longitudinal axis. In the embodiments of the figures, the longitudinal axis of the vessel 2 is horizontal. The vessel 2 could also be slightly inclined from the horizontal plane. The inclination angle of the vessel 2 could be, for instance, in the range of 0-15 degrees relative to the horizontal direction. The vessel 2 could be inclined so that the second end 2b of the vessel 2 is at a lower level than the first end 2a of the vessel 2 to facilitate moving of the organic matter within the vessel 2 from the first end 2a towards the second end 2b, from which the processed organic matter is discharged. However, it is advantageous to arrange the vessel 2 so that its longitudinal axis, which is also the rotation axis of the vessel 2, is horizontal. This reduces forces exerted on the composting apparatus 1 when the vessel 2 is rotated.

The composting apparatus 1 comprises bearings 11, 12 for supporting the vessel 2 and for allowing rotation of the vessel 2. In the embodiments of the figures, the composting apparatus 1 comprises a first bearing arrangement 11 arranged at the first end 2a of the vessel 2 and a second bearing arrangement 12 arranged at the second end 2b of the vessel 2. However, the apparatus 1 could comprise one or more further bearing arrangements. In the embodiment of figures 10 and 11, the first and the second bearing arrangements 11, 12 support the vessel 2 in the vertical and lateral directions. The composting apparatus 1 of figures 10 and 11 is further provided with a guide arrangement 29 that supports the vessel in the longitudinal direction. The guide arrangement 29 comprises a first flange 29a and a second flange 29b surrounding the outer perimeter of the vessel 2 and a rotatable roll 29c arranged between the first flange 29a and the second flange 29b. The guide arrangement 29 is configured to prevent longitudinal displacement of the vessel 2.

The composting apparatus 1 comprises rotating means 23 for rotating the vessel 2. The rotating means 23 can comprise, for instance, an electric, pneumatic or hydraulic motor, or an actuator producing linear movement and means for converting the linear movement into rotation of the vessel 2. The actuator producing linear movement could be, for instance, a hydraulic or pneumatic cylinder or an electric linear actuator. In the embodiment of figures 10 and 11, the rotating means 23 comprise a motor 23a. The first bearing arrangement 11 comprises two rolls 11a, 11b, which are arranged to support the vessel 2. The motor 23a is coupled via a gear 23b and belts 23c to the rolls 11a, 11b for rotating the rolls 11a, 11b. In the embodiment of figures 10 and 11, both rolls 11a, 11b are rotated by the motor 23a, but it could be sufficient to rotate only one of the rolls 11a, 11b. The motor 23a could also be coupled to a roll that is separate from the bearing arrangement 11. The belts 23c can be, for instance, toothed belts or V-belts. Alternatively, the motor 23a could be coupled to the rolls 11a, 11b for instance directly by the gear 23b or by means of a chain. If the motor 23a can be driven with a sufficiently slow speed, it is not necessary to have a gear between the motor 23a and the rolls 11a, 11b.

A feeding opening 3 is arranged at the first end 2a of the vessel 2. Via the feeding opening 3, the organic matter to be processed is fed into the vessel 2. In the embodiment of the figures, the feeding opening 3 is arranged in an end wall of the vessel 2. This allows feeding of the material into the vessel 2 regardless of the angular position of the vessel 2. In the embodiments of the figures, the feeding opening 3 is round. The feeding opening 3 is concentric with the longitudinal center axis of the vessel 2.

A discharge opening 4 is arranged at the second end 2b of the vessel 2. In the embodiment of the figures, the discharge opening 4 is arranged in an end wall of the vessel 4. This allows discharging of the material from the vessel 2 regardless of the angular position of the vessel 2. In the embodiment of the figures, the discharge opening 4 is round. The discharge opening 4 is concentric with the longitudinal center axis of the vessel 2.

In the first embodiment, the composting apparatus 1 is provided with feeding means 13 for feeding the organic matter to be processed into the vessel 2. The feeding means 13 comprise a conveyor. In the first embodiment, the feeding means comprise a screw conveyor 13. The screw conveyor 13 comprises a rotatable screw 14 that is configured to move the material fed into the conveyor forward, a motor 15 for rotating the screw 14, and a hopper 16, through which the material is fed into the conveyor 13. Instead of a screw conveyor, some other type of conveyor could be used as a feeding conveyor. In the first embodiment, the organic matter to be processed is fed into the feeding means 13 using a wheel loader 17. However, the apparatus 1 could be configured to receive the organic matter via another conveyor or directly from a pre-treatment apparatus arranged above the feeding means 13. In figures 10 and 11 feeding means are not shown, but the apparatus 1 of figures 10 and 11 can comprise similar feeding means as the apparatus of the other figures.

The apparatus 1 according to the first embodiment is further provided with discharge means 18 for discharging the processed organic matter from the vessel 2. The discharge means comprise a conveyor 18. In the first embodiment, the discharge means comprise a screw conveyor 18. The screw conveyor 18 comprises a rotatable screw 19 that is configured to move the material fed into the conveyor forward, a motor 20 for rotating the screw 19, and a hopper 21, through which the material is fed into the conveyor 18. In the apparatus of the first embodiment, where the discharge opening 4 is arranged in the end wall of the vessel 2, the hopper 21 of the screw conveyor 18 is arranged within the vessel 2. Instead of a screw conveyor, some other type of conveyor could be used as a discharge conveyor. In figures 10 and 11 discharge means are not shown, but the apparatus of figures 10 and 11 could be provided with similar discharge means as the apparatus according to the first embodiment.

The composting apparatus 1 comprises transfer and mixing elements 5a, 5b, 5c, 5d that are arranged within the vessel 2. The transfer and mixing elements 5a, 5b, 5c, 5d are configured to move the organic matter inside the vessel 2 towards the second end 2b of the vessel 2 and to mix the organic matter in the vessel 2 when the vessel 2 is rotated. The transfer and mixing elements 5a, 5b, 5d, 5d are attached to the inner walls of the vessel 2. The transfer and mixing elements 5a, 5b, 5c, 5d are guide elements, which are shaped and positioned so that as the vessel 2 rotates, the resulting movement of the elements 5a, 5b, 5c, 5d moves the organic matter towards the second end 2b of the vessel 2 and/or mixes the organic matter within the vessel 2. The transfer and mixing elements 5a, 5b, 5c, 5d can be, for instance, steel plates attached to the inner wall of the vessel 2. A transfer and mixing element 5a, 5b, 5c, 5d can be bent and/or it can comprise two or more plates attached to each other. In the embodiments of the figures, the apparatus comprises different types of transfer and mixing elements 5a, 5b, 5c, 5d. Some of the elements are configured to move the organic matter towards the second end 2b of the vessel 2, some of the elements are configured to mix the organic matter, and some of the elements are configured to both mix and move the organic matter. In the embodiments of the figures, the transfer and mixing elements comprise first elements 5a, which are arranged at the first end of the vessel 2. The first elements 5a are configured to move the organic matter forward and to simultaneously mix the material. Second elements 5b are arranged along the longitudinal direction of the vessel 2 and configured to move the organic matter forward. Third elements 5c are also arranged along the longitudinal direction of the vessel 2 and configured to mix the organic matter. Fourth elements 5d are arranged at the second end of the vessel 2 and configured to lift the organic matter into the hopper 21 of the discharge conveyor 18. The organic matter is moved and mixed solely by means of the transfer and mixing elements 5a, 5b, 5c, 5d. The apparatus is thus not provided with any conveyor for moving the organic matter within the vessel 2.

The composting apparatus 1 comprises air supply means for supplying at an aeration zone air into the organic matter to be processed. The aeration zone does not cover in the longitudinal direction of the vessel 2 the whole vessel 2 but only part of the vessel 2. The aeration zone is arranged at the first end 2a of the vessel 2. In the embodiments of the figures, the aeration zone extends from the first end 2a of the vessel 2 to a distance corresponding to approximately 45 percent of the total length of the vessel 2. The aeration zone extends from the first end 2a of the vessel 2 to a distance corresponding to 35-65 percent of the total length of the vessel 2. The term "aeration zone" refers to that area of the vessel 2, at which air can be supplied directly into the organic matter. The aeration zone covering part of the length of the vessel 2 allows composting of the organic matter in two phases. In a first phase, air can be fed into the organic matter via the air supply means. In a second phase, which takes place in a different part of the vessel 2, the composting process continues without air feeding.

In the embodiments of the figures, the aeration zone is configured to cover in the circumferential direction of the vessel 2 approximately half of the vessel 2. The vessel 2 can be configured to be filled to a level of approximately 65-80 % of the diameter of the vessel 2. There is no need to extend the aeration zone around the whole circumference of the vessel 2. The aeration zone extends over at least 120 degrees in the circumferential direction of the vessel 2, or preferably over at least 180 degrees.

In the embodiments of the figures, the air supply means comprise air pipes 8, which are arranged within the vessel 2. Each air pipe 8 comprises a plurality of air outlets 8a. The plurality of air pipes 8 ensure uniform air supply into the organic matter and prevent formation of anaerobic pockets within the organic matter. Full biological degradation of the organic matter is thus ensured. The uniform air supply also helps keeping the metabolic rate of the microorganisms and consequently the temperature of the organic matter within a desired range.

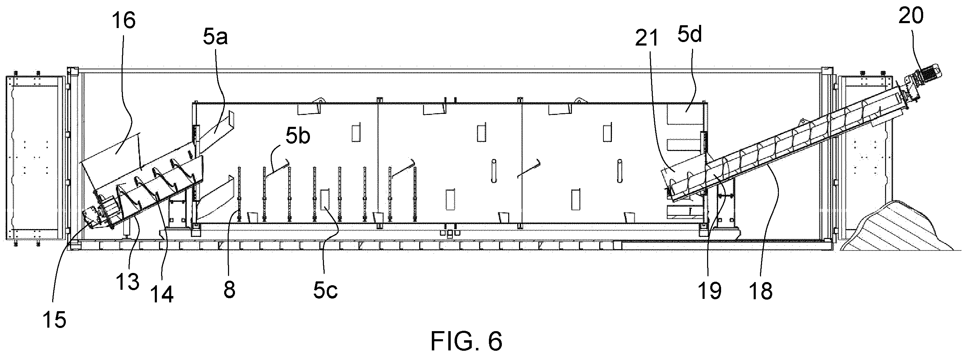

In the embodiment best seen in figures 6, 8 and 9, each air pipe 8 is arranged to follow the inner wall of the vessel 2 in the circumferential direction. Each pipe 8 extends over approximately half of the circumference of the vessel 2. The distance between two adjacent air pipes 8 can be, for instance, in the range of 150-600 mm. The air pipes 8 are elevated from the inner wall of the vessel 2. The air pipes 8 are thus not attached directly to the inner wall of the vessel 2, but there is a gap between the pipe 8 and the inner wall of the vessel 2.The elevation can be, for instance, in the range of 20-100 mm. The air pipes 8 are attached to the inner wall of the vessel 2 by means of fastening elements 28, which are configured to form a gap between the pipe 8 and the inner wall of the vessel 2.

The air pipes 8 are connected to an air manifold 6. Each air pipe 8 is connected to the air manifold 6 by means of a pipe or a hose 7. The air manifold 6 is arranged outside the vessel 2. The air supply means further comprise an air blower 9, which is connected to the air manifold 6.

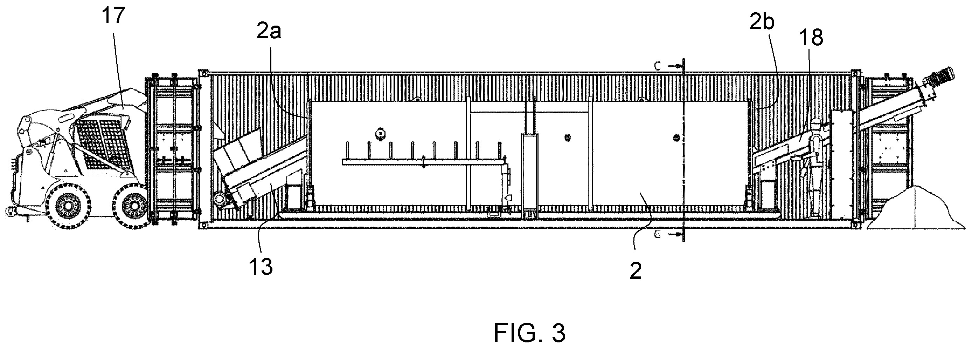

Instead of arranging the air pipes 8 parallel to the circumferential direction of the vessel 2, the air pipes 8 can be parallel to the longitudinal direction of the vessel 2, as shown in figure 11. In the embodiment of figure 11, the air pipes 8 are connected to circumferential pipes 7. A longitudinal pipe 6 functions as an air manifold and supplies air to the circumferential pipes 7. The longitudinal air pipes 8 are connected to the circumferential pipes 7 at both ends, but it would be sufficient to connect the air pipes 8 to a single circumferential pipe 7. Also the circumferential pipes 7 can be provided with air outlets, but that is not necessary. In the embodiment of figure 11, the longitudinal pipe 6 and the circumferential pipes 7 are arranged within the vessel 2. However, the longitudinal pipe 6 and the circumferential pipes 7 could also be arranged outside the vessel 2 in a similar way as the air manifold 6 shown in figures 1 and 3.

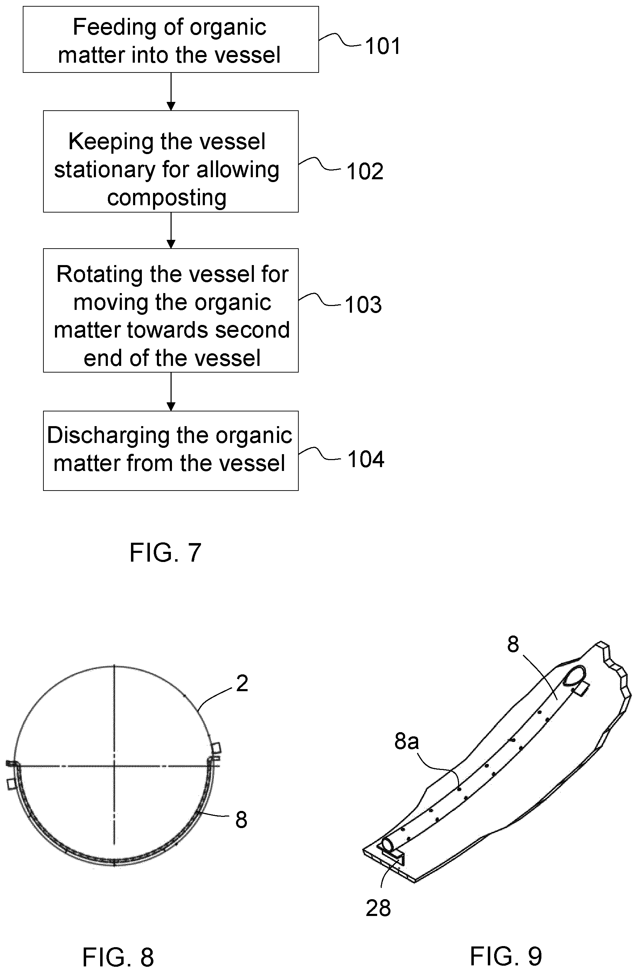

In the embodiment of figure 11, the diameter of the air pipes 8 is smaller than the diameter of the circumferential pipes 7. The circumferential pipes 7 are supported against the inner surface of the vessel 2, and the air pipes 8 are thus elevated from the inner surface. The air pipes 8 could also be elevated from the inner surface in a similar way as in figures 8 and 9.

Instead of the longitudinal or circumferential air pipes 8 shown in the figures, the air pipes could protrude into the vessel in radial direction. However, such an air supply system would be much more complicated to manufacture.

The vessel 2 of the composting apparatus 1 has a resting position, which is shown in the figures. In the resting position, the aeration zone is located in the lower half of the vessel 2. The aeration zone is thus at its lowermost position.

The composting apparatus 1 is further provided with at least one temperature sensor. Figure 2 shows a first temperature sensor 25, which is configured to measure the temperature of the air within the vessel 2. The first temperature sensor 25 is thus located so that when the vessel 2 is in an angular position where the air pipes 8 are covered by the organic matter to be processed, the first temperature sensor 25 is above the organic matter. The first temperature sensor 25 is thus located outside the aeration zone. Figure 2 shows a second temperature sensor 26, which is configured to measure the temperature of the organic matter. The second temperature sensor 26 is located at the aeration zone. In the resting position of the figures, the second temperature sensor 26 is located in the lower half of the vessel 2. The apparatus 1 can comprise more than one first and/or second temperature sensors 25, 26. Especially the temperature of the organic matter can be unevenly distributed, and therefore it may be advantageous to measure the temperature of the organic matter at different locations. The composting apparatus may comprise, for instance, 2-6 second temperature sensors 26.

The composting apparatus 1 comprises at least one humidity sensor 24. The humidity sensor 24 is configured to measure the air humidity within the vessel 2. The humidity sensor 24 is located in a similar way as the first temperature sensor 25. It is thus arranged outside the aeration zone. It is not necessary to arrange the humidity sensor inside the vessel 2, but the humidity sensor can also be arranged in an air discharge duct.

The composting apparatus 1 comprises an air fan 30. The air fan 30 is arranged to control discharge of air from the vessel 2. The air fan 30 can be arranged inside or outside the vessel 2. The air fan 30 can be connected to a separate air outlet. Alternatively, air can be discharged from the vessel via the discharge opening 4 and/or the feeding opening 3. Instead of the air fan 30, discharge of air could be controlled, for instance, by an adjustable flap or other means arranged in an air outlet. However, the air fan 30 ensures effective air discharge.

The composting apparatus 1 comprises means for determining whether the vessel 2 is in the resting position. The means can be, for instance, a switch that is switched from one state to another when the vessel 2 arrives to the resting position. The means could also be a proximity sensor 27. Alternatively, the means could be an angle sensor, which can determine the angular position of the vessel 2.

The composting apparatus 1 can be further provided with many other different sensors and/or switches. The other sensors and switches can comprise, for instance, means for ensuring safety of the composting apparatus. The apparatus 1 could comprise, for instance, sensors for detecting an open inspection hatch or the presence of a person in a danger zone.

The composting apparatus 2 comprises a control unit 22. The control unit 22 is configured to control the operation of different devices of the composting apparatus 1. The control unit 22 controls the operation of the feeding means 13, discharge means 18, air blower 9, rotating means 23 and air fan 30 or other means controlling the discharge of air from the vessel 2. The control unit 22 is also configured to receive data from different sensors and switches of the composting apparatus 1. The control unit 22 can thus receive data from the first and second temperature sensors 25, 26, humidity sensor 24 and proximity sensor 27. The control unit 22 can be configured to control the operation of the different devices based on the data received from the different sensors and switches. The operation of the feeding means 13, discharge means 18, rotating means 13, air blower 9 and air fan 30 can thus be controlled based on the temperature of the organic matter, air temperature within the vessel 2, air humidity within the vessel 2 or angular position of the vessel 2. The operation of the devices could also be controlled based on a combination of different data received from the sensors and/or switches.

The control unit 22 can be connected to the devices and sensors of the composting apparatus 1 either wirelessly or with wired connections. The control unit 22 can be a programmable controller. The control unit 22 could also be a special-purpose computer or a general-purpose computer, such as a PC. The control unit 22 could comprise sub-control units for controlling specific functions of the composting apparatus 1. The same control unit 22 could be used for controlling several composting apparatuses 1. The control unit 22 could also be located remotely.

In a first step 101 of the method according to the invention, i.e. in a feeding step, organic matter is fed into the vessel 2. In a second step 102 of the method, i.e. in a composting step, the vessel 2 is kept stationary for allowing composting of the organic matter. In a third step 103 of the method, i.e. in a moving step, the vessel 2 is rotated for moving the organic matter towards the second end of the vessel 2. In a fourth step 104 of the method, i.e. in a discharging step, the organic matter is discharged from the vessel 2.

The second step 102 of the method forms a composting sequence. The other three steps 101, 103, 104 form a feeding and discharging sequence, where the three steps can take place simultaneously. While new organic matter is fed into the vessel 2 via the first end 2a of the vessel 2, composted organic matter is discharged from the vessel 2 via the second end 2b of the vessel 2, and organic matter within the vessel 2 is moved towards the second end 2b of the vessel 2.

The organic matter to be fed into the composting apparatus 1 may be pretreated. The possible pre-treatment step takes place outside the vessel 2. A pre-treatment step can comprise one or more of the following actions: removing of possible redundant material like metal or plastic, shredding of the organic matter, adding of support material to adjust the moisture, density, and/or the carbon-to-nitrogen ratio of the material to be fed into the composting apparatus, and mixing of the organic matter and the support material. The mixture fed into the vessel 2 of the composting apparatus 1 should typically have a moisture between 60 % and 70 %, a density between 600 kg/m³ and 700 kg/m³ and a C:N ratio between 20 and 30.

In the feeding step 101, the organic matter to be processed is fed into the vessel 2 via the feeding opening 3. The feeding conveyor 13 is operated and the material fed into the hopper 16 of the feeding conveyor 13 is moved by means of the rotating screw 14 into the vessel 2. The rotating screw 14 further mixes the material.

Simultaneously with the operating of the feeding conveyor 13, the discharge conveyor 18 is operated. The discharging step 104 thus takes place simultaneously with the feeding step 101. In the discharging step, material is discharged from the second end 2b of the vessel 2 via the discharge opening 4.

Simultaneously with the operating of the feeding conveyor 13 and the discharge conveyor 18, the vessel 2 is rotated by means of the rotating means 23. As the vessel 2 is rotated, the mixing and transfer elements 5a, 5b, 5c, 5d move the organic matter within the vessel 2 towards the second end 2b of the vessel 2. The moving step 103 thus takes place simultaneously with the feeding step 101 and the discharging step 104.

When the feeding and discharging sequence, which comprises the feeding step 101, the moving step 103 and the discharging step 104, ends, the composting sequence starts. During the composting sequence, the vessel 2 is kept stationary in the resting position. The proximity sensor 27 or other means indicate whether the vessel 2 is in the resting position. If the vessel 2 is not in the resting position, the rotating means 23 are operated to rotate the vessel 2 to the resting position.

According to an embodiment of the invention, the composting step 102 comprises at least two sub-steps. According to an embodiment of the invention, a balancing phase forms a first sub-step. During the balancing phase, the air blower 9 is not operated. The balancing phase continues for a predetermined period of time, for instance for one hour. Alternatively, the balancing phase can continue until a predetermined temperature of the organic matter is reached. The purpose of the balancing phase is to allow the temperature of the organic matter to level out and rise after the feeding step 101. During the balancing phase, the at least one first temperature sensor 25 can monitor the air temperature within the vessel 2 and the at least one second temperature sensor 26 can monitor the temperature of the organic matter at the aeration zone. In addition, the humidity sensor 24 can monitor the air humidity within the vessel 2. The air fan 30 is operated for discharging air from the vessel 2.

According to an embodiment of the invention, during the balancing phase both the temperature of the organic matter and the air humidity within the vessel 2 are monitored, and the discharging of air from the vessel 2 is controlled based on the monitored temperature and air humidity. The controlling of the air discharge can be based on one or more set points for the temperature and the humidity. The speed of the air fan 30 can thus be set based on whether the temperature of the organic matter is below or above a temperature set point and whether the humidity is below or above a humidity set point. Each combination of the measured temperature and humidity values can thus have a corresponding predetermined air fan speed.

Instead of having a group of predetermined air fan speeds for different temperature and humidity levels, the operation of the air fan 30 could be controlled for keeping the humidity close to a humidity target value. The composting apparatus 1 can comprise a PID controller for controlling the operation of the air fan 30. The PID controller can be part of the control unit 22. The PID controller can receive from the humidity sensor 24 a humidity measurement signal, and based on the humidity measurement signal, generate a fan control signal, which is transmitted to the air fan 30 for adjusting the speed of the air fan 30. Alternatively, the operation of the air fan 30 could be controlled by setting a lower limit and an upper limit for the air humidity. The control unit 22 could receive from the humidity sensor 24 a humidity measurement signal, and generate a signal for switching on the air fan 30 when the measured humidity value is above the upper limit, and generate a signal for switching off the air fan 30 when the humidity value is below the lower limit.

During the balancing phase, the temperature of the organic matter should normally steadily rise. The balancing phase is followed by an air supply phase, which forms a second sub-step of the composting step 102. During the air supply phase, air is supplied into the organic matter via the air supply means 6, 7, 8, 9. The air blower 9 is operated for supplying air into the organic matter. Also the air fan 30 is operated for discharging air from the vessel 2. The air supply rate is adjusted on the basis of the temperature of the organic matter at the aeration zone and on the basis of the air humidity within the vessel 2. Also the air discharge rate is adjusted based on the measured temperature of the organic matter at the aeration zone and on the basis of the air humidity within the vessel 2. The aim is to reach a temperature target value in the shortest time possible. The temperature target value can be, for instance, 70 °C.

If the temperature of the organic matter is a lot lower than the temperature target value, the air supply rate is kept low. A low temperature is an indication that the metabolic activity of the bacteria in the vessel 2 must be supported, and the limiting factor for the process speed is the concentration of oxygen, which is needed for the aerobic process. A low but constant air supply rate ensures proper distribution of the oxygen in the organic matter and promotes biological degradation at a high rate.

If the temperature of the organic matter is close to temperature target value, the air supply rate is kept high. High temperature of the organic matter indicates that the aerobic process is reaching its maximum rate and the high temperature coupled with excessive moisture content becomes a limiting factor. The air supplied at a high rate removes the excessive heat and moisture from the organic matter and helps maintaining the high rate of the aerobic process.

For adjusting the air supply rate and the air discharge rate, the temperature of the organic matter is monitored by means of the at least one second temperature sensor 26. A temperature measurement signal from the temperature sensor 26 is transmitted to the control unit 22. In case of several second temperature sensors 26, the control unit 22 can be configured to calculate an average temperature. Also the air humidity is monitored by means of the humidity sensor 24. A humidity measurement signal from the humidity sensor 24 is transmitted to the control unit 22.

Based on the temperature measurement signal or a calculated average temperature and the humidity measurement signal, the control unit 22 generates an air blower control signal and an air fan control signal. The air blower control signal is transmitted to the air blower 9 for controlling the air supply rate and the air fan control signal is transmitted to the air fan 30 for controlling the air discharge rate. The air blower control signal and the air fan control signal can be based on predetermined set points. One or more set points can be determined both for the temperature and the humidity. Certain air blower speeds and air fan speeds can be selected depending on whether the measured humidity and temperature values are below or above the set points.

Alternatively, the air supply rate and the air discharge rate could be controlled steplessly. The control unit 22 could thus be configured to adjust the air supply rate and the air discharge rate steplessly based on the difference between the temperature target value and the measured temperature and on the difference between the humidity target value and the measured humidity.

When the target temperature is reached, the air supply phase is followed by a constant temperature phase, which forms a third sub-step of the composting step 102. In the constant temperature phase, after reaching the temperature target value, the aim is to keep the temperature within a temperature target range for a predetermined period of time. The temperature target range can be, for instance, 68-75 °C. The predetermined period of time can be, for instance, one hour. By keeping the temperature at approximately 70 °C for an hour, hygienization of the organic matter and removal of pathogens is ensured.

Short deviations from the target temperature range may be allowed. For instance, the temperature may be allowed to be outside the target temperature range for at most 5 minutes. If the temperature stays within the target temperature range for the predetermined period for time, or if the deviations from the target temperature range do not exceed the allowed maximum deviations, hygienization of the organic matter is deemed completed.

In the constant temperature phase, the air blower 9 may be switched off when the temperature is within the target temperature phase. The air fan 30 may be operated with a predetermined speed. If the temperature falls below the target temperature range or rises above the range, the air supply rate and the air discharge rate can be controlled based on the measured temperature of the organic matter and the measured air humidity to reach the target temperature range. If the temperature has been outside the target range longer than the allowed maximum time, the counting of the predetermined period is started from zero.

After completion of hygienization, the process may continue with the discharging step and feeding steps. Alternatively, if there is no need to feed new material into the vessel, monitoring of the temperature of the organic matter and the air humidity is continued. The operation of the air blower 9 and the air fan 30 is controlled based on the measured temperature and humidity.

It will be appreciated by a person skilled in the art that the invention is not limited to the embodiments described above, but may vary within the scope of the appended claims.

## Claims

1. A composting apparatus (1) for processing organic matter, the apparatus comprising
- a cylindrical vessel (2) for receiving and processing the organic matter, the cylindrical vessel (2) having a first end (2a) and a second end (2b) and being rotatable about its longitudinal axis,
- a feeding opening (3) arranged at the first end (2a) of the vessel (2) for feeding the organic matter to be processed into the vessel (2),
- a discharge opening (4) arranged at the second end (2b) of the vessel (2) for discharging the processed organic matter from the vessel (2),
- mixing and transfer elements (5a, 5b, 5c, 5d) arranged inside the vessel (2) and being configured to move the organic matter inside the vessel (2) towards the second end (2b) of the vessel (2) and to mix the organic matter when the vessel (2) is rotated, and
- air supply means (6, 7, 8, 9) for supplying air into the organic matter to be processed at an aeration zone of the vessel (2),
**characterised in that** the aeration zone extends in the circumferential direction of the vessel (2) over an angle of at least 120 degrees and in the longitudinal direction of the vessel (2) from the first end (2a) of the vessel (2) to a distance corresponding to 35-65 percent of the total length of the vessel (2).

2. A composting apparatus (1) according to claim 1, wherein the aeration zone extends in the circumferential direction of the vessel (2) over an angle of at least 180 degrees.

3. A composting apparatus (1) according to any of the preceding claims, wherein the air supply means comprise a plurality of air pipes (8) arranged within the vessel (2), each air pipe (8) comprising a plurality of air outlets (8a).

4. A composting apparatus (1) according to claim 3, wherein the air pipes (8) extend in the longitudinal and/or circumferential direction of the vessel (2).

5. A composting apparatus (1) according to claim 3 or 4, wherein the air pipes (8) are elevated 10-100 mm from the inner surface of the vessel (2).

6. A composting apparatus (1) according to any of the preceding claims, wherein the air supply means comprise an air blower (9).

7. A composting apparatus (1) according to any of the preceding claims, wherein the apparatus (1) comprises means (30) for controlling discharging of air from the vessel (2).

8. A composting apparatus (1) according to claim 7, wherein the means for controlling discharging of air from the vessel (2) comprise an air fan (30).

9. A composting apparatus (1) according to any of the preceding claims, wherein the apparatus (1) comprises feeding means (13) for feeding the organic matter to be processed into the vessel (2).

10. A composting apparatus (1) according to any of the preceding claims, wherein the apparatus (1) comprises discharge means (18) for discharging the processed organic matter from the vessel (2).

11. A composting apparatus (1) according to any of the preceding claims, wherein the apparatus (1) comprises temperature measuring means (25, 26) for measuring temperature within the vessel (2) and humidity measuring means (24) for measuring air humidity within the vessel (2), and the apparatus (1) is configured to control air supply via the air supply means (6, 7, 8, 9) and/or discharging of air from the vessel (2) on the basis of at least one the temperature value and at least one humidity value measured within the vessel (2).

12. A method of processing organic matter using a composting apparatus (1) according to any of the preceding claims, the method comprising
- a feeding step (101), in which organic matter is fed into the vessel (2),
- a composting step (102), in which the vessel (2) is kept stationary for allowing composting of the organic matter,
- a moving step (103), in which the vessel (2) is rotated for moving the organic matter towards the second end of the vessel (2), and
- a discharging step (104), in which the organic matter is discharged from the vessel (2).

13. A method according to claim 12, wherein the composting step (102) comprises at least one sub-step, during which
- temperature and humidity in the vessel (2) are monitored, and
- air supply into the vessel (2) via the air supply means (6, 7, 8, 9) and/or discharging of air from the vessel (2) is controlled based on the monitored temperature and the monitored humidity.

## Patentansprüche

1. Kompostiervorrichtung (1) zum Verarbeiten von organischem Material, wobei die Vorrichtung Folgendes umfasst:
- einen zylindrischen Behälter (2) zum Aufnehmen und Verarbeiten des organischen Materials, wobei der zylindrische Behälter (2) ein erstes Ende (2a) und ein zweites Ende (2b) aufweist und um seine Längsachse drehbar ist,
- eine Einfüllöffnung (3), die am ersten Ende (2a) des Behälters (2) angeordnet ist, zum Einfüllen des zu verarbeitenden, organischen Materials in den Behälter (2),
- eine Austragsöffnung (4), die am zweiten Ende (2b) des Behälters (2) angeordnet ist, zum Austragen des verarbeiteten organischen Materials aus dem Behälter (2),
- Misch- und Transferelemente (5a, 5b, 5c, 5d), die im Inneren des Behälters (2) angeordnet und ausgestaltet sind, um das organische Material im Inneren des Behälters (2) hin zum zweiten Ende (2b) des Behälters (2) zu bewegen und das organische Material zu mischen, wenn der Behälter (2) gedreht wird, und
- Luftzuführmittel (6, 7, 8, 9) zum Zuführen von Luft in das zu verarbeitende, organische Material in einer Belüftungszone des Behälters (2),
**dadurch gekennzeichnet, dass** sich die Belüftungszone in der Umfangsrichtung des Behälters (2) über einen Winkel von mindestens 120 Grad und in der Längsrichtung des Behälters (2) vom ersten Ende (2a) des Behälters (2) bis zu einem Abstand erstreckt, der 35-65 Prozent der Gesamtlänge des Behälters (2) entspricht.

2. Kompostiervorrichtung (1) nach Anspruch 1, wobei sich die Belüftungszone in der Umfangsrichtung des Behälters (2) über einen Winkel von mindestens 180 Grad erstreckt.

3. Kompostiervorrichtung (1) nach irgendeinem der vorhergehenden Ansprüche, wobei die Luftzuführmittel eine Vielzahl von Luftrohren (8) umfassen, die innerhalb des Behälters (2) angeordnet sind, wobei jedes Luftrohr (8) eine Vielzahl von Luftauslässen (8a) umfasst.

4. Kompostiervorrichtung (1) nach Anspruch 3, wobei sich die Luftrohre (8) in Längs- und/oder Umfangsrichtung des Behälters (2) erstrecken.

5. Kompostiervorrichtung (1) nach Anspruch 3 oder 4, wobei die Luftrohre (8) 10-100 mm von der Innenoberfläche des Behälters (2) erhöht sind.

6. Kompostiervorrichtung (1) nach irgendeinem der vorhergehenden Ansprüche, wobei die Luftzuführmittel ein Luftgebläse (9) umfassen.

7. Kompostiervorrichtung (1) nach irgendeinem der vorhergehenden Ansprüche, wobei die Vorrichtung (1) Mittel (30) zum Steuern des Ablassens von Luft aus dem Behälter (2) umfasst.

8. Kompostiervorrichtung (1) nach Anspruch 7, wobei die Mittel zum Steuern des Ablassens von Luft aus dem Behälter (2) einen Luftventilator (30) umfassen.

9. Kompostiervorrichtung (1) nach irgendeinem der vorhergehenden Ansprüche, wobei die Vorrichtung (1) Einfüllmittel (13) zum Einfüllen des zu verarbeitenden, organischen Materials in den Behälter (2) umfasst.

10. Kompostiervorrichtung (1) nach irgendeinem der vorhergehenden Ansprüche, wobei die Vorrichtung (1) Austragsmittel (18) zum Austragen des verarbeiteten organischen Materials aus dem Behälter (2) umfasst.

11. Kompostiervorrichtung (1) nach irgendeinem der vorhergehenden Ansprüche, wobei die Vorrichtung (1) Temperaturmessmittel (25, 26) zum Messen der Temperatur innerhalb des Behälters (2) und Feuchtigkeitsmessmittel (24) zum Messen der Luftfeuchtigkeit innerhalb des Behälters (2) umfasst, und die Vorrichtung (1) ausgestaltet ist, um die Luftzufuhr über die Luftzuführmittel (6, 7, 8, 9) und/oder das Ablassen von Luft aus dem Behälter (2) auf der Basis von mindestens einem Temperaturwert und mindestens einem Feuchtigkeitswert, die innerhalb des Behälters (2) gemessen werden, zu steuern.

12. Verfahren zum Verarbeiten von organischem Material unter Verwendung einer Kompostiervorrichtung (1) nach irgendeinem der vorhergehenden Ansprüche, wobei das Verfahren Folgendes umfasst
- einen Einfüllschritt (101), bei dem organisches Material in den Behälter (2) eingefüllt wird,
- einen Kompostierschritt (102), bei dem der Behälter (2) stationär gehalten wird, zum Ermöglichen des Kompostierens des organischen Materials,
- einen Bewegungsschritt (103), bei dem der Behälter (2) gedreht wird, zum Bewegen des organischen Materials hin zum zweiten Ende des Behälters (2), und
- einen Austragsschritt (104), bei dem das organische Material aus dem Behälter (2) ausgetragen wird.

13. Verfahren nach Anspruch 12, wobei der Kompostierschritt (102) mindestens einen Teilschritt umfasst, während dessen
- Temperatur und Feuchtigkeit im Behälter (2) überwacht werden, und
- die Luftzufuhr in den Behälter (2) über die Luftzuführmittel (6, 7, 8, 9) und/oder das Ablassen von Luft aus dem Behälter (2) basierend auf der überwachten Temperatur und der überwachten Feuchtigkeit gesteuert wird.

## Revendications

1. Appareil de compostage (1) destiné au traitement des matières organiques, l'appareil comprenant
- une cuve cylindrique (2) permettant de recevoir et de traiter la matière organique, la cuve cylindrique (2) ayant une première extrémité (2a) et une seconde extrémité (2b) et pouvant tourner autour de son axe longitudinal,
- une ouverture d'alimentation (3) agencée au niveau de la première extrémité (2a) de la cuve (2) pour alimenter la cuve (2) en matière organique à traiter,
- une ouverture de décharge (4) agencée au niveau de la seconde extrémité (2b) de la cuve (2) pour décharger la matière organique traitée du récipient (2),
- des éléments de mélange et de transfert (5a, 5b, 5c, 5d) agencés à l'intérieur de la cuve (2) et étant conçus pour déplacer la matière organique à l'intérieur de la cuve (2) vers la seconde extrémité (2b) de la cuve (2) et pour mélanger la matière organique lorsque la cuve (2) est mise en rotation, et
- des moyens d'alimentation en air (6, 7, 8, 9) pour fournir de l'air dans la matière organique à traiter au niveau d'une zone d'aération de la cuve (2),
**caractérisé en ce que** la zone d'aération s'étend dans la direction circonférentielle de la cuve (2) sur un angle d'au moins 120 degrés et dans la direction longitudinale de la cuve (2) de la première extrémité (2a) de la cuve (2) à une distance correspondant à 35-65 pour cent de la longueur totale de la cuve (2).

2. Appareil de compostage (1) selon la revendication 1, dans lequel la zone d'aération s'étend dans la direction circonférentielle de la cuve (2) sur un angle d'au moins 180 degrés.

3. Appareil de compostage (1) selon l'une quelconque des revendications précédentes, dans lequel les moyens d'alimentation en air comprennent une pluralité de tuyaux d'air (8) agencés à l'intérieur de la cuve (2), chaque tuyau d'air (8) comprenant une pluralité de sorties d'air (8a).

4. Appareil de compostage (1) selon la revendication 3, dans lequel les tuyaux d'air (8) s'étendent dans la direction longitudinale et/ou circonférentielle de la cuve (2).

5. Appareil de compostage (1) selon la revendication 3 ou 4, dans lequel les tuyaux d'air (8) sont surélevés de 10 à 100 mm par rapport à la surface intérieure de la cuve (2).

6. Appareil de compostage (1) selon l'une quelconque des revendications précédentes, dans lequel les moyens d'alimentation en air comprennent une soufflerie d'air (9).

7. Appareil de compostage (1) selon l'une quelconque des revendications précédentes, dans lequel l'appareil (1) comprend des moyens (30) permettant de commander l'évacuation de l'air de la cuve (2).

8. Appareil de compostage (1) selon la revendication 7, dans lequel les moyens permettant de commander l'évacuation de l'air de la cuve (2) comprennent un ventilateur d'air (30).

9. Appareil de compostage (1) selon l'une quelconque des revendications précédentes, dans lequel l'appareil (1) comprend des moyens d'alimentation (13) pour alimenter la cuve (2) en matière organique à traiter.

10. Appareil de compostage (1) selon l'une quelconque des revendications précédentes, dans lequel l'appareil (1) comprend des moyens de décharge (18) pour décharger la matière organique traitée de la cuve (2).

11. Appareil de compostage (1) selon l'une quelconque des revendications précédentes, dans lequel l'appareil (1) comprend des moyens de mesure de la température (25, 26) pour mesurer la température à l'intérieur de la cuve (2) et des moyens de mesure de l'humidité (24) pour mesurer l'humidité de l'air à l'intérieur de la cuve (2), et l'appareil (1) est configuré pour commander l'alimentation en air par l'intermédiaire des moyens d'alimentation en air (6, 7, 8, 9) et/ou l'évacuation de l'air de la cuve (2) sur la base d'au moins la valeur de température et d'au moins une valeur d'humidité mesurées à l'intérieur de la cuve (2).

12. Procédé de traitement de la matière organique à l'aide d'un appareil de compostage (1) selon l'une quelconque des revendications précédentes, le procédé comprenant
- une étape d'alimentation (101), dans laquelle la matière organique est alimentée dans la cuve (2),
- une étape de compostage (102), dans laquelle la cuve (2) est maintenue immobile pour permettre le compostage de la matière organique,
- une étape de déplacement (103), dans laquelle la cuve (2) est mise en rotation pour déplacer la matière organique vers la seconde extrémité de la cuve (2), et
- une étape de déchargement (104), dans laquelle la matière organique est déchargée de la cuve (2).

13. Procédé selon la revendication 12, dans lequel l'étape de compostage (102) comprend au moins une sous-étape, au cours de laquelle
- la température et l'humidité dans la cuve (2) sont surveillées, et
- l'alimentation en air de la cuve (2) par l'intermédiaire des moyens d'alimentation en air (6, 7, 8, 9) et/ou l'évacuation de l'air de la cuve (2) sont commandées en fonction de la température et de l'humidité surveillées.
